# EUROPEAN PATENT APPLICATION

(11) **EP 3 692 893 A1**
(43) Date of publication of application: **12.08.2020**
(21) Application number: 19155423.7
(22) Date of filing: 05.02.2019
(51) Int. Cl.: A61B 5/00, A61B 8/12, A61B 8/00, B06B 1/06, A61M 25/00, A61M 25/01, A61M 25/09

(54) **SENSOR HAVING AN ADAPTED HOUSING**

(71) Applicant: Koninklijke Philips N.V., 5656 AG Eindhoven (NL)
(72) Inventor: WEEKAMP, Johannes Wilhelmus, 5656 AE Eindhoven (NL); VAN DE MOLENGRAAF, Roland Alexander, 5656 AE Eindhoven (NL); VAN DER HORST, Arjen, 5656 AE Eindhoven (NL)
(74) Representative: de Haan, Poul Erik

(57) **Abstract**

The present invention relates to a sensor comprising a housing; at least a sensor element arranged within the housing; plurality of elongate structures providing one or more slots; a front end and a back end provided by the housing; wherein the one or more slots are arranged between the front end and the back end of the housing; and wherein the one or more fingers are configured to receive the sensor element extending within the one or more slots. Furthermore, a device comprising the sensor and a method for manufacturing the sensor is provided.

## Description

### FIELD OF THE INVENTION

The present invention relates to a sensor, a device comprising the sensor and a method for manufacturing the sensor.

### BACKGROUND OF THE INVENTION

Measuring blood flow in arteries will help physicians in making the right diagnoses for proper treatment. The measuring principle can be based on the Doppler effect. The actuator/receiver for such a device can be based on a circular disk of piezo electric material. This is the current state of the art for intravascular flow velocity sensing. Another application that makes use of the piezo electric material is for localization of sensors on devices in an ultrasound field (e.g. Philips OnVision). There also the disk transducers can be used. A piezo disk based device might however have limited sensitivity.

The tip of a flow sensor guidewire usually has a rounded tubular shape to be atraumatic. Square or rectangular shaped transducers fit less optimally in a round tube compared to round (circular disk) transducers. When placing a square or rectangular shaped transducer into a round housing, the dicing size should be compact enough to fit the housing. When inserted into a round tubular guidewire a square or rectangular transducer provides a reduction in active surface compared to a round transducer. This leads to a diminished acoustic pressure that the transducer is able to generate. On the other hand, fabrication of square or rectangular shaped transducers is quite efficient, which is due to the dicing process for separation of the individual transducer elements.

US 2017/0065225A1 describes a pressure sensing guidewire for measuring blood pressure. The guidewire has a tubular member with a pressure sensor (e.g. piezoelectric or ultrasound pressure sensor) arranged therein. A thinned inner wall of the tubular member or an increased inner diameter defines a housing in the tubular member to accommodate the pressure sensor therein. A plurality of slots are provided on the tubular member allowing a body fluid to flow from a position along the exterior of the guidewire through the slots into the lumen of the tubular member where the pressure sensors are exposed to the fluid. The pressure sensor is arranged on a landing region free of slots so that the pressure sensor is not deformed by the fluid pressure.

### SUMMARY OF THE INVENTION

There is thus a need to provide a medical device with improved sensitivity of the acoustic pressure output of the sensor arranged therein.

The object of the present invention is solved by the subject-matter of the independent claims; further embodiments are incorporated in the dependent claims. It should be noted that the following described aspects of the invention apply also for the sensor, for the device comprising the sensor and for the method for manufacturing the sensor.

According to the present invention, a sensor comprises a housing, at least a sensor element arranged within in the housing, one or more fingers providing one or more slots and a front end and a back end provided by the housing. The one or more slots are arranged between the front end and the back end of the housing. The one or more fingers are configured to receive the sensor element extending through the one or more slots.

The sensor comprises sensor element(s) for (Doppler) flow measurement, ultrasound imaging or other sensor elements used in medical devices. Ultrasound tracking of the sensor by receiving signals from an external ultrasound probe is also provided. The housing comprises a metal material.

A sensor element comprises sensor types such as an ultrasound transducer element, ultrasound emitter/sensor element, a pressure sensor, an image sensor, additionally or alternatively also a temperature sensor, pH sensor or biomarker sensor or other type of sensors is comprised by a sensor element. Furthermore, the sensor element can comprise a combination of the mentioned sensor types.

A sensor element can comprise an acoustic stack of materials comprising an active part, which generates ultrasound waves upon an electrical drive signal, and passive parts for coupling acoustic waves only into the desired medium. The passive parts comprise backing material for attenuating transmission of ultrasound waves in undesired directions (e.g. proximal shaft of the device), and matching layers for efficiently coupling ultrasound waves into a medium in the desired direction (e.g. anatomical structures, air, etc.). The sensor element(s) may originate from a conventional piezoelectric ultrasound emitter/sensor array or from a micro-machined ultrasound emitter/sensor array (capacitive or piezoelectric). The sensor element(s) may comprise multiple or single acoustic stack of materials. Ultrasound emitter/sensor elements or transducer sensor elements provide an increased aperture for receiving ultrasound scattering and reflection from anatomical structures upon impinging ultrasound waves.

The sensor, e.g. a tip of a flow guide sensor wire, may usually have a round tubular shape to be atraumatic, so that damage at the inside of the arteries is prohibited. The sensor provides improved sensitivity due to the increased active surface area of the sensor element arranged therein.

For sensor elements comprising an acoustic stack of materials, e.g. a backing layer, a piezo material (single crystal or sintered structure) and a matching layer, the sensitivity of the sensor element is improved. However, such sensor element or stacked sensor element usually comprise a rectangular or square shape, due to separation of larger stacks into single sensor elements or multiple (stacked) sensor elements. Other shapes like a hexagon or octagon made by dicing are also comprised by the sensor element described herewith.

The non-round shape of the sensor element is due to the fact that the different layers are bonded in large sizes (typical 1 cm*6 cm) and are separated after bonding by dicing into single elements (typical 250 micron*250 micron). Also the dicing needs to be along the axes of the crystal structure, so that the sensitivity of the sensor is not affected resulting in non-round shaped sensors. The size of the sensor element will determine the output power (performance) and the sensitivity for the reflected signal. In other words, the sensor size is an important factor in determining the performance and sensitivity for the reflected signal.

A rectangular, square or non-round sensor shape might not fit optimally in a round tubular shaped housing. For providing improved fit of the sensor, the housing comprises one or more elongate structures, which may also be called fingers. The finger(s) are configured to receive the sensor element. When the sensor element, particularly a rectangular or square sensor element, is received, it is held in the housing by the fingers. In other words, the sensor element abuts the fingers.

The elongate structures or fingers provide one or more slots which are arranged between the front end and the back end of the housing. The finger(s) can be provided with a curved outer shape. The inner shape of the finger(s), i.e. the inner shape abutting the sensor, can be provided as a flat or curved shape.

The back end can be provided with a coil for improved flexibility of the sensor. The housing is connected to the coil by soldering welding or gluing. If no coil is provided, the housing can be connected to a core wire by soldering or gluing. A core wire will always be provided with or without coil, so that the tip of the sensor is pre-shaped or shaped during use for better navigation.

When received in the housing, the sensor element, particularly a rectangular or square sensor element, extends through the one or more slots. In other words, one or more corners of the sensor element extend through the one or more slots.

Furthermore, due to the one or more slots arranged between the front end and the back end of the housing, the fitting of the sensor element, particularly a square or rectangular sensor element, can be significantly improved thus leading to a sensor with improved sensitivity.

According to an example, the sensor element is received in a maximal size fitting manner in the housing. In other words, the size of the sensor element is substantially the same as the size of the housing. Particularly, the diagonal diameter of a non-round sensor element, e.g. square or rectangular shape, is substantially the same as the outer diameter of the housing. All or at least a part of the corners of the sensor element are arranged on a circumference of the inner or outer wall of the housing. This is due to the corners of the sensor element extending through the slots. In an example, the corners of a square sensor element will extend within four slots of the housing so that all or at least a part of the corners lie on the circumference between the inner and outer wall of the housing. In alternative example, maximal size fitting manner means the diameter of the housing and the diagonal of the sensor element are substantially the same.

A typical outer diameter (OD) of a sensor housing is usually approximately 0.35 mm. Taking this as an example, the maximum outer diameter (OD) of a round transducer received in the housing is approximately 0.3 mm. The specific surface area of such round transducer is 0.07 mm². Fitting a squared sensor in such housing, where the fitting is as in the prior art, the housing having the inner diameter of approximately 0.30 mm, the maximum specific surface area is 0.045 mm². Compared to the mentioned specific surface area of a round transducer (of 0.07 mm²), this leads to a reduction of 36% of the significant surface area of the squared sensor (fitted as in the prior art).

By providing a sensor, where the sensor element is received in the housing comprising one or more slots, a maximum sized non-round sensor will fit inside the housing. Taking the example of the squared sensor in a housing with an OD of 0.35 mm, as mentioned above, the gain in specific surface area can increase up to 20%.

Thus, a maximal size fitting manner of the sensor element in the housing is achieved. For example, the outer diameter of the sensor housing is 350 micron and the diagonal diameter of the sensor element is also substantially 350 micron (of course including measurement tolerance). A tight fitting between the sensor element and the housing is thus achieved. Therefore, a (significant) reduction in the specific surface area of the sensor element compared with round sensors elements, as in the prior art, is avoided.

According to an example the housing comprises an open front end and the one or more fingers are arranged at the back end of the housing. In other words, the sensor is provided in the shape of a fork tip. Each of the finger(s) provides a free end at the open front end of the housing. The finger(s) are fixed to the back end or are an integral part of the back end of the housing.

In an example, one or more slots fully or partially extend from the front end to the back end of the housing. The length of the slot(s) or the length of the finger(s) can be adapted to the required length of the to be received sensor element during manufacturing. The length of the one or more slots is larger or substantially equal to the length of the received sensor element.

According to an example, the housing comprises at least two parts. The two part housing provides easy assembly of the sensor element. Also, an improved atraumatic shape is provided.

In an example, a first part comprises an open front end providing a ring-shaped edge and the one or more fingers are arranged at the ring-shaped edge. Such first part of the two-part housing provides an improved traumatic shape of the sensor. The fingers are attached or integrally formed with the edge provided in the shape of a ring or circular shape.

In an example, a second part comprises the back end providing indentations for receiving the one or more fingers. In other words, the fingers engage with the indentations thereby forming the housing of the sensor. The fingers can be permanently or temporarily fixed in the indentations. These fingers can be fixed by glue, welding or mechanical fitting (e.g. interference fit).

In an example, the at least two parts are arranged symmetrically and each comprises half of an open front end, one or more fingers and half of the back end. In other words, the sensor is provided in two symmetric parts which are arranged face to face. The symmetry plane extends along a longitudinal axis of the housing or of the sensor. After receiving the sensor element, the two parts form the housing comprising an open front end having a ring-shaped edge, one or more slots and the back end. The back end can be disk-shaped.

According to an example, the housing comprises a round tubular shape. Such housing provides a sensor with a traumatic shape.

According to the present invention, also a device comprising a medical interventional device and the sensor is provided, wherein the sensor is provided at the medical interventional device or may be provided as an integral part thereof. Thus, the device may also be referred to as medical device. The sensor can be provided at a tip or end of the medical interventional device. The medical interventional device comprises an intravascular guidewire, a catheter, interventional needle or other similar devices used for diagnosis and treatment.

According to an example, the device is used to assess blood flow velocity or for acquisition of image data. The sensor element comprises at least a pressure sensor for measuring the blood flow velocity. In other examples, the sensor element comprises at least an image sensor for acquisition of image data, e.g. of a vessel. A maximal size fitting of the sensor element, i.e. the flow sensor, in the sensor housing is provided, thus increasing the active surface of the sensor and improving the performance and sensitivity thereof.

According to an example, the sensor housing provides exposure of both a front face and side areas of the sensor for ultrasound tracking by an external ultrasound probe. For ultrasound localization (e.g. Philips OnVision), an ultrasound transducer designed for imaging or flow measurement is usually exposed on its front side, according to prior art devices. If such transducer is used for ultrasound localization, then the signal strength can be limited when the transducer is facing away from the (external) ultrasound source.

A sensor with a housing, providing one or more slots, provides reducing such orientation sensitivity for transducers during localization, as both the front face and the side areas of the sensor / sensor element are exposed to a beam of the external ultrasound probe.

According to an aspect of the present invention, also a method for manufacturing a sensor is provided. The method comprises: a) providing a housing; b) forming one or more elongate structures (fingers) configured to provide one or more slots; c) providing the housing with a front end and a back end; d) arranging at least one ultrasound sensor element within the housing; wherein the one or more slots are arranged between the front end and the back end of the housing; and wherein the one or more fingers are configured to receive the sensor extending through the one or more slots.

The housing can be provided as metal housing and can be laser cut to obtain one or more finger(s) in which the sensor element is positioned. Also, the one or more finger(s) can be provided by welding or gluing the finger(s) to the front or back end of the housing.

According to an example, a shrink tube as permanent or temporary mold is applied to the housing. The shrinktube can either be used as a temporary mold for the potting or gluing material or can be an integral part of the device with the benefit of an additional shielding and improved atraumaticy.

According to an example, the sensor is formed by casting for providing atraumatic properties. This also provides the benefit of an additional shielding and improved atraumaticy.

In any of the embodiments the material used to fill the slots and form the distal end of the sensor is preferably translucent to acoustical waves, in particular to ultrasound waves.

The present invention provides adapting the housing of the sensor where slots are made, so that the size of the rectangular/square sensor element is maximized (a maximum size sensor element fits into the housing having a required limited size). After applying a thin shrink tube or a temporary mold, the shape of the sensor tip will be formed by casting, in order to be atraumatic. Taking for example the square shaped sensor in the housing with an internal diameter of 0.3 mm, the gain in active surface area is up to 20%.

An additional problem solved by the inventive concept relates only to the ultrasound localization (e.g. OnVision). An ultrasound transducer designed for imaging or flow measurement is usually only exposed on its front side. If such transducer is used for ultrasound localization, then the signal strength will be very limited when the transducer is facing away from the (external) ultrasound source. A more exposed open transducer (sensor) housing helps reducing this orientation sensitivity for transducers (sensors) that are used simultaneously for physiological parameter measurements (flow and/or pressure) and localization.

Thus, an increasing active surface area related to acoustic pressure output is provided while ultrasound localization by reducing orientation sensitivity is improved.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1: an embodiment of a housing of a sensor in a front perspective view;
Fig. 2: the embodiment of Fig. 1 in a rear perspective view;
Fig. 3: a further embodiment of a housing of a sensor;
Fig. 4A-C: an embodiment of a sensor shown in different assembling steps;
Fig. 5A-B: a further embodiment of a sensor;
Fig. 6A-C: a further embodiment of a sensor shown in different assembling steps;
Fig. 7A-C: a further embodiment of a sensor shown in different assembling steps;
Fig. 8: an embodiment of a system comprising the sensor assembled in a device;
Fig. 9: a flow chart of a method for manufacturing of a sensor;
Fig. 10: a surface area of a sensor element fitted as in the prior art.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

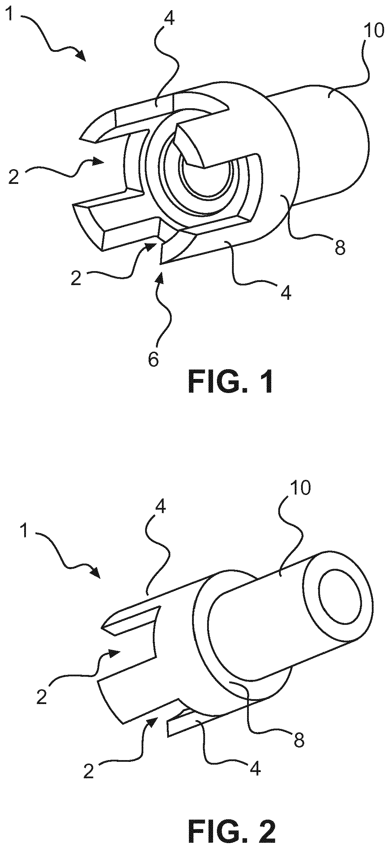

Fig. 1 shows exemplarily and schematically an embodiment of a housing 1 of a sensor in a front perspective view. The housing 1 comprises four elongate structures (fingers) 4 forming four slots 2. The slots 2 are arranged between a front end 6 and a back end 8 of the housing. The housing comprises a support structure 10 that facilitates connection of the housing to elements of an interventional device, such as core wire.

The four fingers 4 are configured to receive a sensor element extending through the one or more slots 2 (this is also shown in more detail in the figures showing the sensor). In this embodiment the fingers 4 are provided with a slanted edge, so that the inner face of the fingers 4 is smaller than the outer face. The outer shape of the fingers 4 is curved. The inner shape of the fingers 4 abutting the sensor element may be flat or curved.

The housing 1 is provided with an open front end 6 and the one or more fingers 4 are arranged at the back end 8 of the housing. The housing 1 is provided in the shape of a fork tip. Each of the fingers 4 provides a free end at the open front end 6 of the housing 1. The fingers 4 are provided as an integral part of the back end 8 of the housing 1. The fingers 4 may also be fixed to the back end 8 by gluing, welding or mechanical fitting.

The housing 1 can be provided as metal housing and can be laser cut to obtain the fingers 4 in which the sensor element is positioned. Also, the one or more fingers can be provided by welding or gluing the fingers 4 to the front 6 or back end 8 of the housing 1.

The one or more slots 2 fully extend from the front end 6 to the back end 8 of the housing 1. The length of the slots 2 or the length of the fingers 4 can be adapted to the required length of the to be received sensor element during manufacturing. The length of the slots 2 is substantially equal to the length of the received sensor element. In other embodiments the slots 2 may partially extend from the back end 8 of the housing 1. The length of the slots can also be larger than the received sensor element.

Fig. 2 shows the embodiment of Fig. 1 in a rear perspective view, wherein the arrangement of the support structure 10 at the back end 8 is shown. As in Fig. 1 the back end 8 is a disk-shaped. In other embodiments also other shapes of the back end 8 are possible. A core wire of an interventional device (e.g. guidewire) can be attached to the back end 8 by gluing, welding or mechanical fitting. In some embodiments the back end 8 may be the support structure 10.

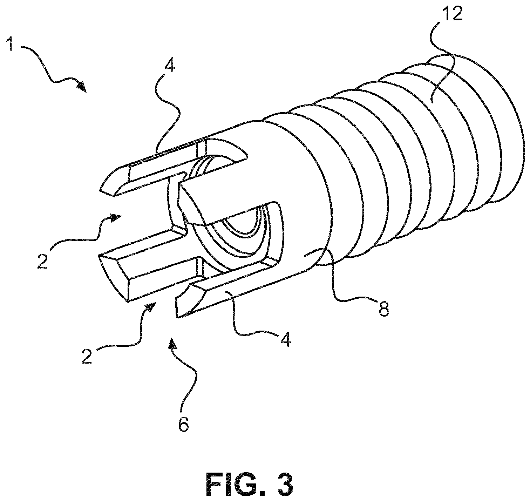

Fig. 3 shows exemplarily and schematically a further embodiment of a housing 1 of a sensor. The housing 1 comprises the four elongate structures (fingers) 4 comprising four slots 2. The slots 2 are arranged between a front end 6 and a back end 8 of the housing. A coil 12 is arranged at the back end 8 of the housing 1 for improved flexibility of the sensor housing and/or its connection to the interventional device. The coil 12 may replace or be considered as the support structure 10. The housing is connected to the coil 12 by soldering, welding, gluing or mechanical fitting.

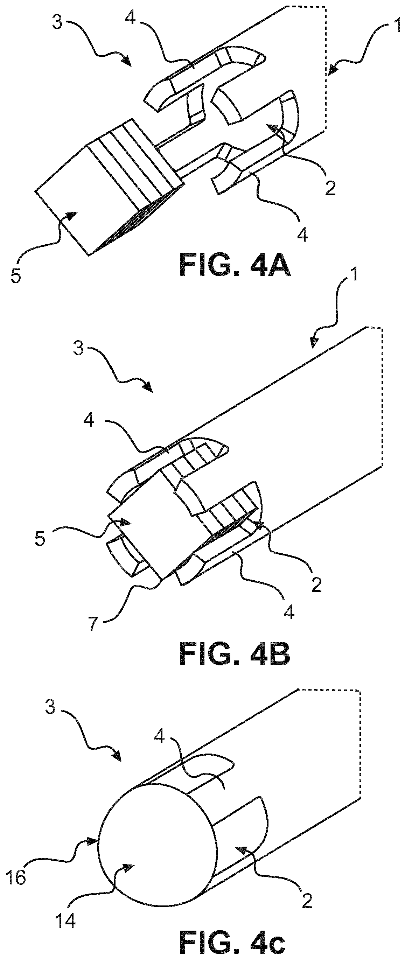

Figs. 4A-C show exemplarily and schematically an embodiment of a sensor 3 in different assembly steps. Figure 4A shows the sensor 3 before a sensor element 5 is received within the housing 1. The sensor element 5 comprises an acoustic stack. Other types of sensor elements 5 may be received by the housing 1. The housing 1 is as described in Figs. 1-3.

Fig. 4B shows the sensor element 5 received within the fingers 4 of the housing 1. The sensor element 5 has four corners 7 which extend radially within the four slots 2. As shown in Fig. 4B the sensor element 5 abuts the fingers 4 of the housing 1.

The sensor element 5 fits in a maximal size fitting manner in the housing 1. In an example, the outer diameter of the sensor housing 1 is 350 micron and the diagonal diameter of the sensor element is also substantially 350 micron (evidently, all dimensions are including measurement tolerance). A tight fitting between the sensor element 5 and the housing 1 can be achieved by suitable dimensioning of the elongate structures (fingers). Therefore, a (significant) reduction in the specific surface area of the sensor element 5 compared with round sensors elements, as in the prior art, is avoided.

Fig. 4C shows the sensor 3 with a masterbond casting which has been applied in a temporary mold 16. This also provides the benefit of an additional shielding and improved atraumaticy. The casting can also be provided by a shrink tube 14.

As can be seen in Fig. 4C the slots 2 are filled after casting and are evenly aligned, with other words being flush, with the elongate structures (fingers) 4. The tip of the sensor 3 comprises a round seamless shape after casting. Thus, the atraumaticy of the sensor 3 is improved. The material used to fill the slots and form the distal end of the sensor is preferably translucent to acoustical waves, in particular to ultrasound waves.

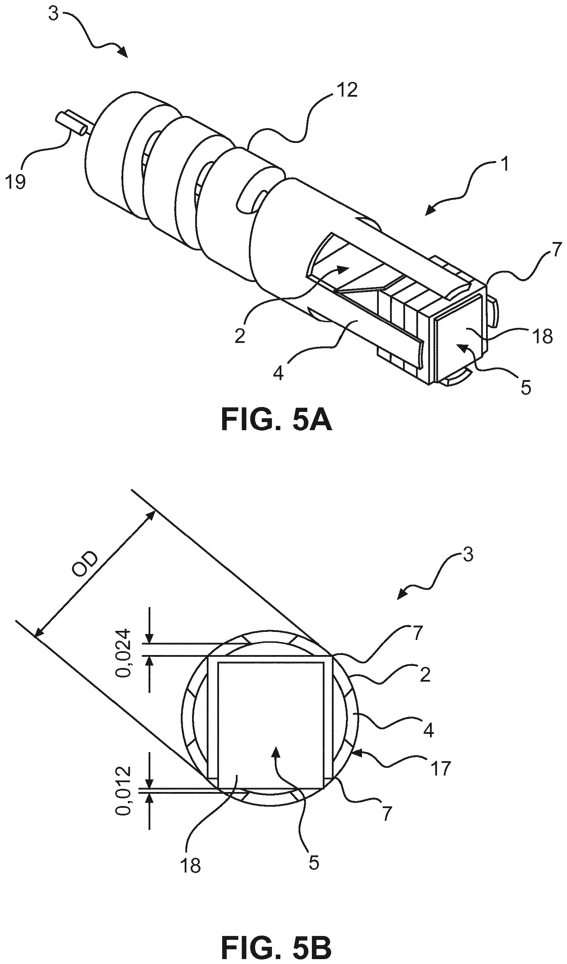

Fig. 5A shows exemplarily and schematically a further embodiment of a sensor 3 comprising a housing 1. The housing 1 comprises four elongate structures (fingers) 4 connected to a back end 8 and four slots 2 providing the characteristics as described above (which are not repeated in detail for this embodiment).

The sensor element 5 partially extends along the length of the fingers 4 and the length of the slots 2. The corners 7 of the sensor element 5 extend through the slots 2. The sensor element 5 is provided with an interconnect 18 for electrical connection of the sensor, which is wrapped around the sensor element 5.

The housing 1 of the sensor 3 is integrally formed with a coil 12. Bifilar wires 19 may be provided which extend through the coil 12. The bifilar wires 19 are connected to the interconnect 18.

Fig. 5B shows a front view of the sensor 3. The following description of the front view also applies for the other embodiments of the sensor 3 with or without an interconnect and is described here in more detail.

The interconnect 18 is in front of the sensor element 5. The corners 7 of the sensor element 5 extend through the slots 2. The sensor element 5 is received in a maximal size fitting manner in the housing 1. The size of the sensor element 5 is substantially the same as the size of the housing 1. The diagonal diameter of the square sensor element 5 is substantially the same as the outer diameter OD of the housing 1.

The corners 7 of the sensor element 5 are arranged on a circumference of the outer wall 17 of the housing 1. This is due to the corners 7 of the sensor element 5 extending through the slots 2. A maximal size fitting manner of the sensor element 5 is thus provided, so that the size of the housing 1 and the sensor element 5 are substantially the same. In other embodiments the corners 7 of the sensor element 5 might be arranged such that at least a part of the corners 7 are arranged between the outer and inner wall circumference of the housing.

The sensor element 5 is arranged in the housing 1 in a loose fit manner. In other words, the sensor element 5 abuts the fingers 4 of the sensor housing including a gap therebetween. The gap between the sensor element 5 and the fingers 4 is about 12 micron or 24 micron in this embodiment. In other embodiments larger or smaller sizes of the gap are possible.

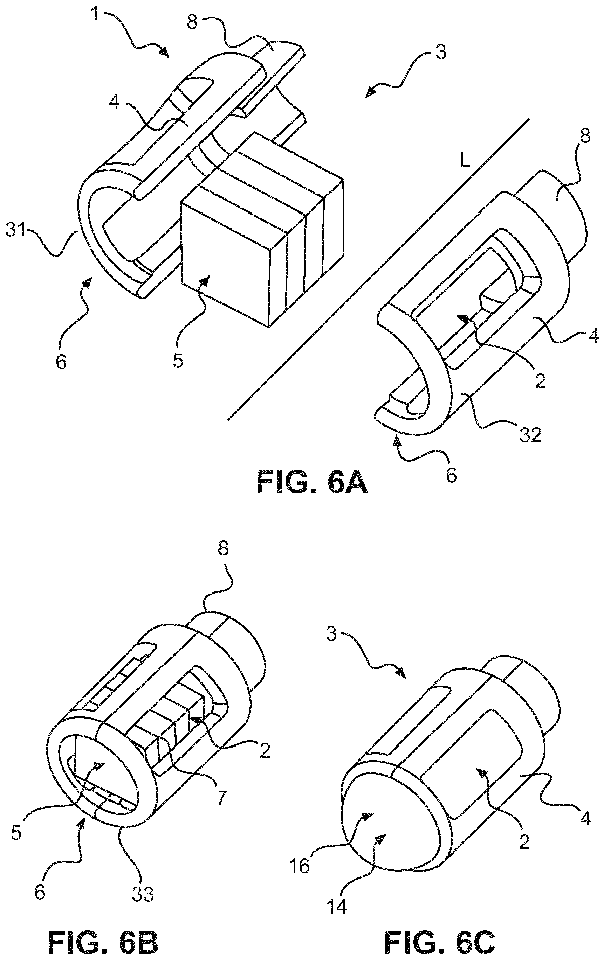

Figs. 6A-C show exemplarily and schematically a further embodiment of a sensor 3 in different assembling steps. The housing 1 comprises two parts 31, 32. The two part housing 1 provides easy assembly of the sensor element 5. Also, an improved atraumatic shape is provided.

The two parts 31, 32 are arranged symmetrically. Each part 31, 32 comprises half of an open front end 6, three elongate structures 4 and half of the back end 8. The housing is provided in two symmetric parts 31, 32 which are arranged opposite to each other. The symmetry plane extends along a longitudinal axis L of the housing or of the sensor. Each of the parts 31, 32 is provided as one-part design, e.g. the front end 6, the elongate structures 4 and the back end 8 are provided as an integral component.

Fig. 6B shows the sensor element 5 received in the housing 1. The two parts 31, 32 form the housing 1 comprising an open front end 6 having a ring-shaped edge 33, slots 2 and a tubular shaped back end 8. The back end 8 can also be disk-shaped.

The corners 7 of the sensor element 5 extend radially within the slots 2 and substantially align with the inner or outer diameter of the housing 1 (as also described in Fig. 5B).

Fig. 6C shows the sensor 3 with a masterbond casting which has been applied in a temporary mold 16. This also provides the benefit of an additional shielding and improved atraumaticy. The casting can also be provided by a shrink tube 14.

As can be seen in Fig. 6C the slots 2 are filled after casting and are evenly aligned with the fingers 4. The tip of the sensor 3 comprises a round shape after casting. Thus, the sensitivity of the sensor 3 is improved by providing a maximal size fitting manner of the sensor element 5 in the housing 1, while the ring-shaped edge 33 facilitates casting the atraumatic distal tip of the sensor.

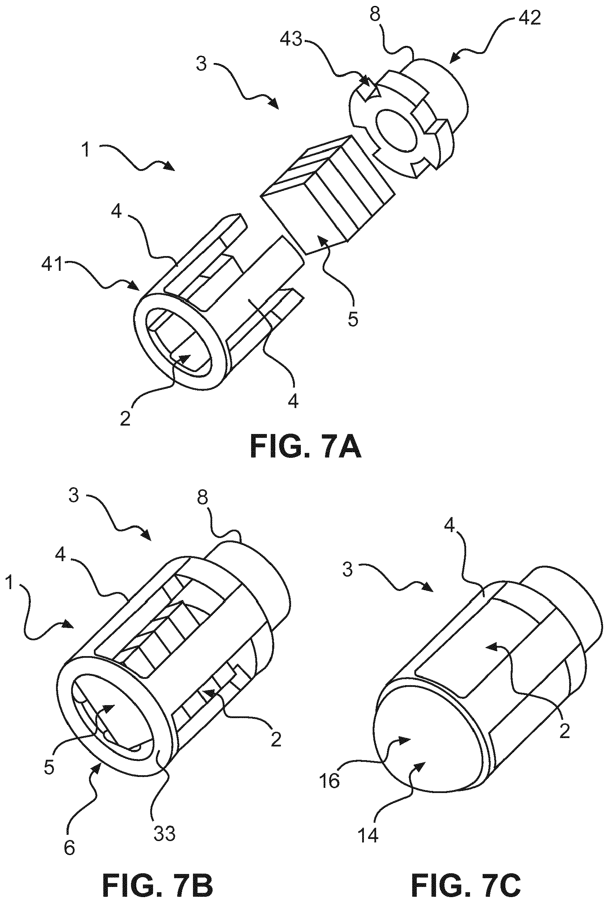

Figs. 7A-C show exemplarily and schematically a further embodiment of a sensor 3 in different assembly steps.

The housing 1 comprises two parts 41, 42. The two part housing 1 provides easy assembly of the sensor element 5. Also, an improved atraumatic shape is provided.

The first part 41 comprises an open front end 6 providing a ring-shaped edge 33 and elongate structures (fingers) 4 are arranged at the ring-shaped edge 33. Thus, the first part 41 of the two-part housing 1 provides a support for an improved traumatic shape of the sensor 3. The fingers 4 are attached or integrally formed with the edge 33 provided in the shape of a ring or circular shape.

The second part 42 comprises the back end 8 providing indentations 43 for receiving the fingers 4. The fingers 4 engage with the indentations 43 thereby forming the housing 1 of the sensor 3. The fingers 4 can be permanently or temporarily fixed in the indentations 43. The fingers 4 can be fixated by glue, welding or mechanical fitting.

Fig. 7B shows the sensor element 5 received in the housing 1. The two parts 41, 42 form the housing 1 comprising an open front end 6 having a ring-shaped edge 33, slots 2 and a back end 8 which is disk-shaped. The corners 7 of the sensor element 5 extend radially within the slots 2 and substantially align with the inner or outer diameter of the housing 1 (as also described in Fig. 5B).

Fig. 7C shows the sensor 3 with a masterbond casting which has been applied in a temporary mold 16. This also provides the benefit of an additional shielding and improved atraumaticy. The casting can also be provided by a shrink tube 14.

As can be seen in Fig. 7C the slots 2 are filled after casting and are evenly aligned with the fingers 4. The tip of the sensor 3 comprises a round shape after casting. Thus, the atraumaticy of the sensor 3 is improved by providing a support for casting of the atraumatic tip by the ring shaped distal edge 33 of the housing, and the sensitivity of the sensor is improved by a maximal size fitting manner of the sensor element 5 in the housing 1.

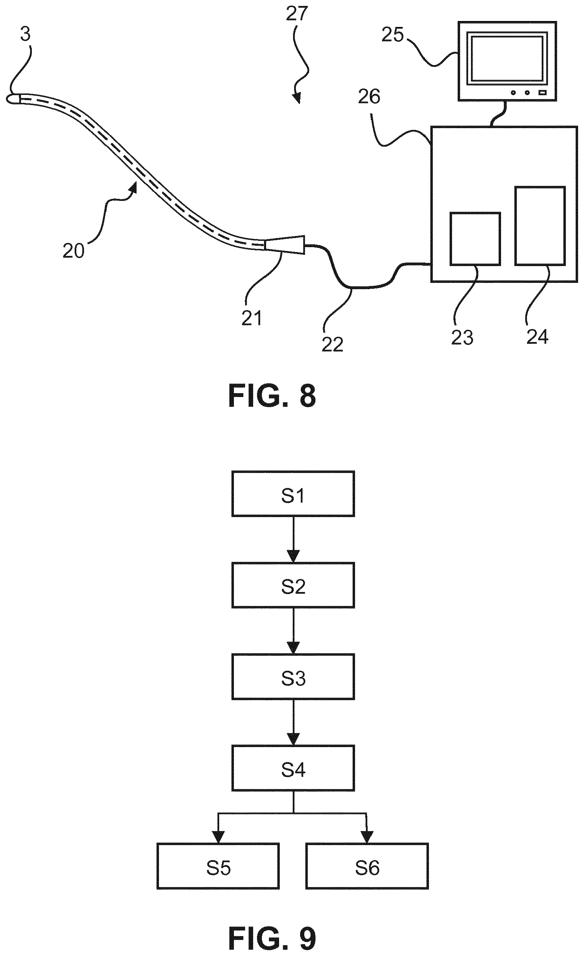

Fig. 8 shows exemplarily and schematically an embodiment of a system 27 according to the invention. The system 27 comprises a sensor 3 in a medical interventional device 20. The sensor 3 is provided in the medical interventional device 20 or as integral part thereof.

In embodiments the medical interventional device 20 can be operatively coupled to an apparatus 26. The apparatus 26 can be connected to a display unit 25 for displaying measurement information, e.g. ultrasound measurement information, collected with the device 20.

The apparatus 26 can be arranged to send a signal to at least a sensor element 5 integrated into or attached to the medical interventional device 20 through a transmission path 22 and to receive a detection signal from the sensor element 5 through the same transmission path 22. The apparatus 26 further comprises a processor 23, which is operable to process detection signals from the sensor element 5 of the medical interventional device 20. The apparatus 26 comprises an internal memory unit 24 for storing data resulting from processing of detection signals.

The elongate body of the device 20 can comprise a proximal portion 21 for coupling the device 20 to the apparatus 26 through transmission path 22. The proximal end 21 of the device 20 may directly be coupled to the apparatus 26 without the transmission path 22. Alternatively, the device 20 may be connected wirelessly to the apparatus 26 and/or display 25, by incorporating an emitter-receiver in the proximal portion 21 of the device

The medical interventional device 20 comprises intravascular guidewire, catheters, interventional needles or other similar devices used for diagnosis and treatment. The housing of the sensor 3 provides exposure of both the front face and the side areas of the sensor for ultrasound tracking by an external ultrasound probe (not shown). When exposed to the ultrasound of the external probe, the sensor 3 with a housing 1, providing one or more slots 2, reduced orientation sensitivity for ultrasound sensor elements or transducers during localization, as the front face and the side areas of the sensor 3 (sensor element 5) are exposed to a beam of the external ultrasound probe.

The apparatus 26 is configured to detect the location of the ultrasound sensor 3 with respect to the external ultrasound probe based on time of flight measurement, or from path detection of interference pattern created by simultaneous emission of ultrasound waves from the ultrasound sensor 3 and the external ultrasound probe. The location detection of the ultrasound sensor 3 with respect to the external ultrasound probe can be accomplished simultaneously with measurement of the at least one of the flow velocity measurement, the imaging of appearance of anatomy and the volumetric flow, due to the increased sensitivity of the sensor 3 by exposure of its side areas through the slots.

Fig. 9 shows a flow chart of a method for manufacturing of a sensor 3. The method comprises the steps of providing S1 a housing 1 and forming S2 one or more elongate structures (fingers) 4 configured to provide one or more slots 2. The housing is provided S3 with a front end 6 and a back end 8. At least one sensor element 5 is arranged S4 within the housing.

One or more slots 2 are arranged between the front end 6 and the back end 8 of the housing 1. The one or more fingers 4 are configured to receive the sensor element extending through the one or more slots 2.

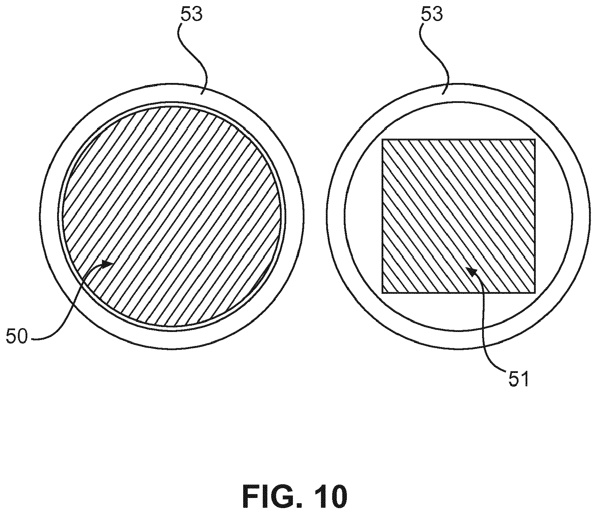

Fig. 10 shows schematically a surface area of a sensor element fitted as in the prior art. The tip of a flow sensor guidewire is shown as a rounded tubular shape 53. The square shaped transducer 51 fits less optimally in the round tube 53 compared to the round (circular disk) transducer 50.

The dicing size of the square shaped transducer 51 is compact enough to fit the tubular shape 53 of the housing. When inserted into a round tubular guidewire a square transducer 51 provides a reduction in active surface compared to a round transducer 50. Fig. 10 illustrates the loss in specific surface of the squared transducer 51 versus the round transducer 50 in the same size housing.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated, and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A sensor, comprising:
- a housing (1) comprising a plurality of elongate structures (4) extending between a front end (6) and a back end (8) of the housing, wherein the elongate structures form one or more slots (2) between the front end and the back end; and
- a sensor element (5) arranged within the housing;
wherein at least a portion of the sensor element radially extends within the one or more slots.

2. Sensor according to claim 1, wherein a diagonal of the sensor element is substantially identical with an outer diameter of the housing.

3. Sensor according to claim 1 or 2, wherein the housing comprises an open front end and the plurality of elongate structures longitudinally extend from a back end and are arranged on a back end structure of the housing.

4. Sensor according to claim 3, wherein the housing comprises an annular structure (33) forming the open front end, the plurality of elongate structures longitudinally extend from and are arranged on the annular structure.

5. Sensor according to claim 4, wherein the housing is assembled from two parts (31, 32) symmetrical with respect to a longitudinal plane comprising the longitudinal axis of the housing, each part comprising half of the annular structure and one or more of the plurality of the elongate structures.

6. Sensor according to claim 1 or 2, wherein the housing comprises an annular structure (33) forming an open front end, the plurality of elongate structures longitudinally extend from the front end toward the back end and are arranged on the annular structure, forming a first part (41) of the housing, and wherein a back end structure forming a second part (42) of the housing comprises indentations (43) for receiving the plurality of elongate structures.

7. Sensor of any of the preceding claims, wherein the sensor comprises a filling material at the front end and in the one or more slots, providing an atraumatic frontal and lateral surface of the sensor.

8. Sensor of claim 7, wherein the material is translucent to acoustic waves.

9. Sensor of any of the preceding claims, wherein the sensor is adapted at the back end for electrical and mechanical connection to an interventional medical device.

10. An interventional medical device (20), comprising a sensor (3) according to any of the claims 1 to 9, wherein the sensor is provided on the interventional device or as an integral part thereof.

11. Device according to claim 10, wherein the sensor is an ultrasound sensor, adapted for at least one of a flow velocity measurement, imaging of appearance of anatomy, volumetric flow measurement, and assessment of a location of the ultrasound sensor with respect to an ultrasound source.

12. A system (27), comprising:
a device according to claim 11;
a display unit (25)
an apparatus (26) configured to receive measurement signals from the sensor of the device, to process the measurement signals from the sensor into output information, and to output the output information to the display unit.

13. System according to claim 12, wherein the apparatus is configured to detect the location of the ultrasound sensor with respect to the ultrasound source, simultaneously with measurement of the at least one of the flow velocity measurement, the imaging of appearance of anatomy and the volumetric flow measurement.

14. A method for manufacturing a sensor (3), comprising the following steps:
providing a housing (1) comprising a plurality of elongate structures (4) extending between a front end (6) and a back end (8) of the housing, the elongate structures forming one or more slots (2) between the front end and the back end;
arranging a sensor element (5) within the housing;
wherein at least a portion of the sensor element radially extends within the one or more slots.

15. Method according to claim 14, further comprising:
forming atraumatic frontal and lateral surfaces of the sensor by adding a material to the front end and into the slots, adjacent to the plurality of elongate structures and the sensor element.
